# EUROPEAN PATENT SPECIFICATION

(11) **EP 3 313 508 B1**
(45) Date of publication and mention of the grant of the patent: **29.04.2020**
(21) Application number: 16734121.3
(22) Date of filing: 22.06.2016
(51) Int. Cl.: A61N 5/06

(54) **LIGHT THERAPY BANDAGE SYSTEM**
LICHTTHERAPIE-VERBANDSSYSTEM
SYSTÈME DE BANDAGE POUR LUMINOTHÉRAPIE

(30) Priority: 23.06.2015 US 201514747687
(43) Date of publication of application: 02.05.2018
(73) Proprietor: Johnson & Johnson Consumer Inc., Skillman, NJ 08558 (US)
(72) Inventor: TAPPER, Jay, Skillman, NJ 08558 (US); SHUTER, David, Skillman, NJ 08558 (US); ALTHOFF, Charles, Peter, Skillman, NJ 08558 (US); MICHAELSON, Jeff, Skillman, NJ 08558 (US); CRADDOCK, Bradley, Feild, Skillman, NJ 08558 (US); DING, Lulin, Skillman, NJ 08558 (US); VIVANT, Marc-Auelien, Skillman, NJ 08558 (US)
(74) Representative: Carpmaels & Ransford LLP
(86) International application number: PCT/US2016/038606
(87) International publication number: WO 2016/209856

(56) References cited:
- EP-A1- 0 412 629
- WO-A1-98/06456
- WO-A1-2011/112931
- WO-A1-2015/069627
- US-A1- 2003 009 205
- US-A1- 2005 177 093
- US-A1- 2006 173 514
- US-A1- 2007 208 395
- US-A1- 2014 072 932
- US-A1- 2014 277 298
- US-A1- 2015 165 231

## Description

### FIELD

The present embodiments relate to devices for delivering light-based skin therapy treatments for improving skin health, and/or relieving subdermal tissue using light-emitting diode (LED) light therapy, although other types of light radiating sources can be used.

### BACKGROUND

Certain light spectrums emitted by LEDs (blue or red) are known to be therapeutic for skin treatment by being beneficial to better factor wound healing or relieving muscular or other subdermal tissue pain. However, there is a need to provide users/patients with a convenient at-home light therapy delivery device such as a wearable bandage that is adjustable or flexible to conform to different sizes and shapes, and that is simple to use without user discomfort. The alternative is visiting a doctor's office to receive treatments.

Prior known light therapy devices have suffered from problems relating to the exposure of the LEDs and the associated circuitry to power the LEDs to contact by users. More particularly, in an effort to maximize light communication to a patient, the LEDs have been disposed in a manner which allow them to be physically engaged (e.g., touched) by a patient, or even contact a treatment surface, which processes are debilitating to the LEDs as a result of the accumulation of dirt and oil. In addition, any such engagement can be potentially dangerous to patients who are exposed to the sharp or hot edges of the LEDs and the associated circuitry. The exposure of detailed circuitry presents an intimidating and unpleasant experience.

Another problem with prior known devices is that the LED arrangement is fixed and not adjustable to better correspond to wound location, size or shape, or to be better placed relative to pain areas. The LEDs of such devices are not selectively arrangeable in a variety of patterns to better enable the application of the device near particular pain areas of a wound.

US 2006/173514 describes a light emitting treatment device including one or more light members, which are configured to emit light energy for the purpose of performing localized photodynamic therapy at a targeted field. The light members may be disposed in a substantially uniform array and be configured to emit energy in a substantially uniform pattern. The light treatment device has a self-contained energy supply. The light emitting treatment device may be controlled to deliver one or more various light doses and dose rates at various light frequencies per treatment. The treatment device may be made of a polymeric material configured to conform to a body surface.

US 2007/208395 describes an apparatus for treating a target body surface using a radiation applicator. The therapeutic treatment apparatus adapted to conform to a patient's body. The treatment apparatus comprises a plurality of light sources coupled with a flexible substrate, a light integrator in at least a portion of the optical path between the light source and the patient's body surface, a power supply, and a controller.

US 2014/277298 describes a radiant energy bandage system including a plurality of therapeutic lamps and a controller for operating the lamps. Batteries power the lamps and are secured to a flexible fabric layer supporting the lamps and the controller.

It is desired to provide alternative means of using the benefits of the light therapy in a manner to maximize therapeutic efficiencies in exposure while maintaining ease and convenience of use. For this reason, a variety of light weight, flexible and adjustable embodiments are disclosed within this disclosure incorporating a variety of energy varying applications responsive to user conditions or needs.

### SUMMARY

The invention is defined by the claims. According to an exemplary embodiment of this disclosure, provided, a phototherapy system and device includes a therapeutic lamp platform for radiant lamps such as LEDs which are disposed in an assembly comprising a multi-layer structure wherein the LEDs are guarded from patient contact.

The exemplary embodiments disclosed provide an adjustable/flexible platform for providing a light-based therapy that is adaptable to the user's receptive surfaces, i.e., treatment areas, whether based on size or condition, wherein the light therapy can be applied without limitation of the kind of light and without limitation of the ultimate purpose of the therapy, i.e., beauty, health, pain relief and/or wound healing. Such sources can vary in the form of the radiant energy delivery. Pulsed light (IPL), focused light (lasers) and other methods of manipulating light energy are encompassed within the presently disclosed embodiments. Other methods of light emission may include continuous, pulsed, focused, diffuse, multi wavelength, single wavelength, visible and/or non-visible light wavelengths.

According to an exemplary embodiment of this disclosure, forms such as a shaped/fitted bandage with LED light emitted from LED bulbs or LED strips that are capable of being adjusted to accommodate variances in a desired treatment area.

According to one exemplary embodiment of this disclosure, a phototherapy device is provided which includes a stretchable and/or flexible wearable therapeutic lamp platform including a plurality of radiant lamps configured to provide radiant energy to a user treatment area; a stretchable and/or flexible reflective wall including a plurality of radiant energy communication areas aligned with the radiant lamps and disposed to communicate the radiant energy to the user treatment area; and a stretchable and/or flexible adhesive layer including a first surface and a second surface, the first surface removably attached to the reflective layer and the second surface operatively associated with removably attaching the wearable therapeutic lamp platform to the user treatment area.

According to another exemplary embodiment of this disclosure, provided is a stretchable and/or flexible wearable phototherapy device including a plurality of radiant energy pods, each pod including one or more radiant lamps to provide radiant energy to a user treatment area, and each pod stretchably and flexible connected to one or more other pods; and a control pod stretchably and flexibly connected to one or more radiant energy section, the control pod operatively connected to the radiant energy pods and configured to control an operation of the radiant lamps.

The present disclosure thus describes a fully stretchable and/or flexible and adjustable LED device which provides improved usability and light dispersion. Such a device includes a light therapy bandage system including a spacing and/or insulating layer to effectively elevate the lamp radiation from the patient's treatment area (e.g. skin). According to one exemplary embodiment, the lamps are recessed relative to the insulating layer and further covered by a sheer mesh layer to protect the user from being able to contact the lamps. Moreover, the disclosed embodiments may or may not be used with lotions, creams and/or ointments which enhance the efficacy of the delivered phototherapy radiation to provide treatment to a user treatment area.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIGURE 1A is a plan view of an exemplary embodiment of a therapeutic lamp platform including a lumbar brace according to this disclosure;
FIGURE 1B is an opposite plan view of the therapeutic lamp platform of FIG. 1A;
FIGURE 2 is an exploded view of the therapeutic lamp platform shown in FIGS. 1A and 1B;
FIGURE 3 is a perspective view of the device shown in FIGS. 1A and 1B on a patient;
FIGURE 4A is a perspective view of another exemplary embodiment of a wearable therapeutic lamp platform including a knee brace according to this disclosure;
FIGURE 4B is an alternative view of the device of FIG. 4A;
FIGURE 4C is an alternative view of the device of FIG. 4A;
FIGURE 4D is an alternative view of the device of FIG. 4A;
FIGURE 5 is an exploded perspective view of the device of FIG. 4A;
FIGURE 6 is an alternative exemplary embodiment of a knee brace according to this disclosure;
FIGURE 7 is an exploded view of the device of FIG. 6;
FIGURE 8 is another embodiment of a knee brace;
FIGURE 9 is another embodiment of a knee brace ;
FIGURE 10 is a top perspective view of one embodiment of the subject bandage system;
FIGURE 11 is a bottom view of the device of FIG. 10;
FIGURE 12 is an exploded view of the device of FIG. 10;
FIGURES 13A and 13B illustrate a stretchable and bendable wearable therapeutic lamp platform according to an exemplary embodiment of this disclosure;
FIGURE 14 is an exploded view of the stretchable and bendable wearable therapeutic lamp platform illustrated in FIGS. 13A and 13B;
FIGURE 15 is a plan view of the reflective layer as shown in FIG. 14;
FIGURE 16 is a plan view of the stretchable and bendable top layer as shown in FIG. 14;
FIGURE 17 is a plan view of the stretchable and bendable bottom layer as shown in FIG. 14;
FIGURE 18 shows a layout of LED strips according to an exemplary embodiment of this disclosure as shown in FIG. 14;
FIGURE 19 is an enlarged detail view of the heating component and temperature sensor(s) arrangement as shown in FIG. 14;
FIGURE 20 is an enlarged detail view of the circuit board, i.e., controller, and the battery arrangement as shown in FIG. 14;
FIGURE 21 is an enlarged detail view of the bottom casing for housing the circuit board and batteries as shown in FIG. 14;
FIGURE 22 is an enlarged detail view of the top casing for housing the circuit board and batteries as shown in FIG. 14;
FIGURES 23A and 23B illustrate an exemplary embodiment of a flexible wearable therapeutic lamp platform according to an exemplary embodiment of this disclosure;
FIGURE 24 is an exploded view of the flexible wearable therapeutic lamp platform illustrated in FIGS. 23A and 23B;
FIGURE 25 is a plan view of the reflective layer as shown in FIG. 24, without the LED clearance holes shown;
FIGURE 26 is a plan view of the biocompatible sticky gel layer as shown in FIG. 24;
FIGURE 27 is a plan view of the flexible layer as shown in FIG. 24;
FIGURE 28 is a plan view of the bendable bottom layer as shown in FIG. 24;
FIGURE 29 shows a layout of LED strips according to an exemplary embodiment of this disclosure as shown in FIG. 14;
FIGURE 30 is an enlarged detail view of the heating component and temperature sensor(s) arrangement as shown in FIG. 24;
FIGURE 31 is an enlarged detail view of the circuit board, i.e., controller, and the battery arrangement as shown in FIG. 24;
FIGURE 32 shows a process to replace an adhesive layer on a wearable therapeutic lamp platform according to an exemplary embodiment of this disclosure;
FIGURE 33 shows a process to apply a wearable therapeutic lamp platform to a user treatment area according to an exemplary embodiment of this disclosure;
FIGURES 34A and 34B illustrate another stretchable wearable therapeutic lamp platform according to an exemplary embodiment of this disclosure; the therapeutic lamp platform including a SIM (Subscriber Identity Module) operatively connected to the platform;
FIGURES 35A and 35B illustrate an exemplary embodiment of a wearable therapeutic lamp platform including a SIM top end card slot;
FIGURES 36A and 36B illustrate an exemplary embodiment of a wearable therapeutic lamp platform including a SIM side card slot;
FIGURES 37A and 37B illustrate an exemplary embodiment of a wearable therapeutic lamp platform including a SIM retro video game card slot;
FIGURES 38A, 38B and 38C illustrate an exemplary embodiment of a wearable therapeutic lamp platform including a combination SIM and SIM card reader;
FIGURES 39A, 39B and 39C illustrate an exemplary embodiment of a wearable therapeutic lamp platform including a combination SIM and SIM card reader outside of the main pod;
FIGURES 40A, 40B and 40C illustrate of a stretchable wearable lamp platform according to an exemplary embodiment of this disclosure;
FIGURE 41 is a perspective view of an exemplary embodiment of another stretchable wearable lamp platform according to this disclosure;
FIGURE 42 illustrates the adhesive layer construction of the stretchable wearable lamp platform shown in FIG. 41;
FIGURES 43A and 43B are detail views of the structural and electrical interconnection of the pods associated with the stretchable wearable lamp platform shown in FIG. 41;
FIGURES 44A and 44B are additional detail views of the structural and electrical interconnections of the pods associated with the stretchable wearable lamp platform shown in FIG. 41;
FIGURES 45A, 45B and 45C illustrate a flexible wearable lamp platform according to an exemplary embodiment of this disclosure;
FIGURE 46 illustrates the flexible wearable lamp platform shown in FIGS. 45A, 45B and 45C, applied to a user's lower back area;
FIGURE 47 illustrates a flexible wearable lamp platform according to an exemplary embodiment of this disclosure;
FIGURE 48 illustrates an exemplary embodiment of the replaceable adhesive layers included in the flexible wearable lamp platform shown in FIG. 47;
FIGURE 49 illustrates the layered construction of the replaceable adhesive layers shown in FIG. 48; and
FIGURES 50A-50E are flow charts of a control program to operate a flexible wearable lamp platform according to an exemplary embodiment of this disclosure.

### DETAILED DESCRIPTION

The subject embodiments relate to a phototherapy system including methods and devices, preferably comprising a wearable device integrated with a portable battery pack for powering therapeutic lamps in the device. The subject devices display numerous benefits including a light platform wherein the platform and the lamps therein are properly positionable relative to a user treatment area during use, where no human touch is required during treatment. That is, structural componentry of the device not only supports the lamp platform on the user, but functions as a guide for the appropriate disposition of the lamps relative to the treatment areas of the user. The structural assembly of the device precludes sharp or hot surfaces from being engageable by a user as the lamps are recessed relative to an inner reflective surface nearer to and facing the patient treatment surface. Circuit componentry to communicate power to the lamps is also encased within a flexible and stretchable wall structure. Therapeutic light, shining through wall radiant energy communication areas, such as, but not limited to, apertures, mesh and clear/translucent layers, is communicated to the user while the lamps and the circuitry are effectively covered within the layered wall structure. A surface is thus presented to the user that is properly spaced for the desired therapeutic treatments, yet provides improved ventilation so that an aesthetic and appealing device surface is presented to the user that minimizes user discomfort. Other benefits relate to the adjustability of the device in the form of a bandage which forms upon user receipt to match a treatment surface, e.g., back or knee, of the user. The overall assembly is purposefully constructed of relatively light weight and minimized componentry for ease of user use and comfort.

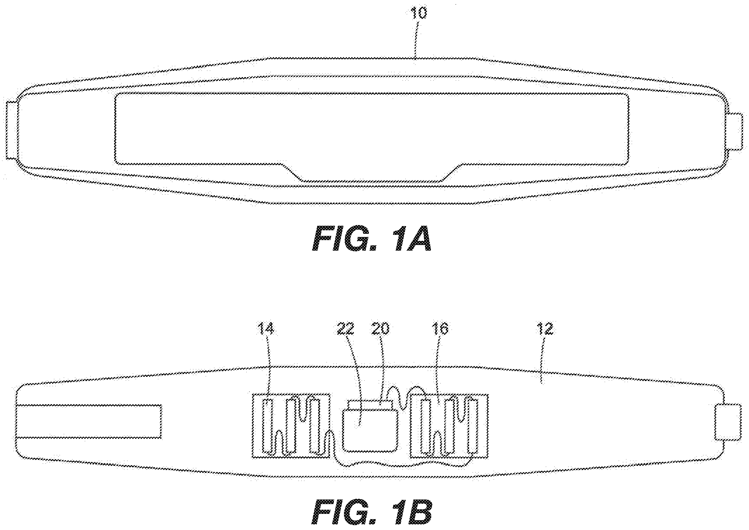

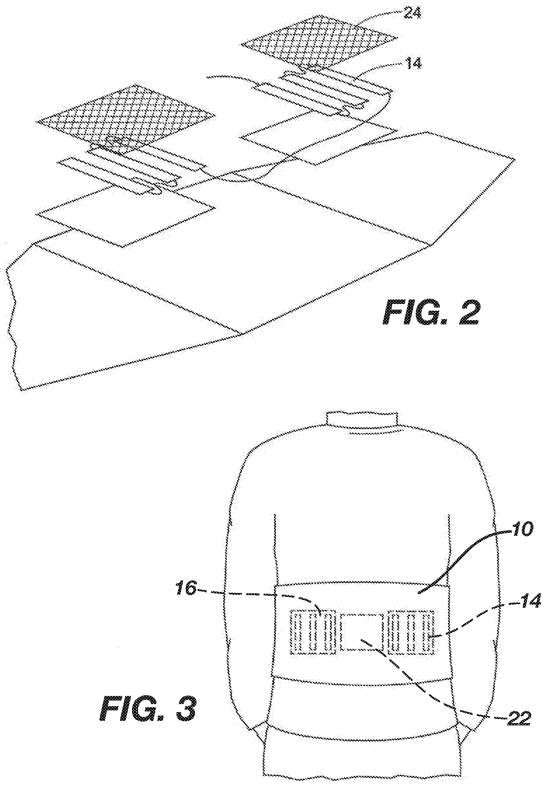

More particularly, and with reference to FIGS. 1A, 1B and 2, an exemplary embodiment is shown including a lumbar brace 10 that can be worn by a patient/user such as shown in FIG. 3. The brace 10 can be supported and affixed on the user by a hook-and-loop locking fabric at the terminal ends of the brace. Such a brace can include heat wraps for lower back and hips 14 on the exterior of the brace 10 opposite of the patient facing surface. The LED platform of the bandage includes an elastic member 12 on which LED strips 14 are mounted on a support layer 16 that is heat insular and/or reflective. It is important that the layer 16 be flexible and stretchable with the elastic bandage 12. Note that the wires connecting the LEDs to the battery pouch 22 are of extra length to allow stretching of the dimension between the LED strips. Power is supplied by a battery pack 20 received in battery pouch 22. The LED lights 14 are spaced from direct engagement of the patient by an insular layer 24 which can range from a mesh cloth to a flexible sheet of formable material in which the strips are integrally molded.

In one exemplary embodiment, the mesh cloth allows communication of the lamp radiation through to the patient without reflection.

In another exemplary embodiment, the flexible formable material 24 has apertures (not shown) functioning as a window to allow the light to pass through and the remainder of the material 24 includes a light reflective surface. In this embodiment, the LEDs are effectively hidden from the patient, where layer 24 is a mesh cloth where the patient can see the LEDs tips and the associated circuitry.

The subject system may also include control systems to vary light intensity, frequency or direction. A portable battery pack is integrated to the wearable phototherapy device, and may include a removable replaceable battery pack or a rechargeable battery pack.

The subject adjustability can be implemented through "smart" processing and sensor systems for enhanced flexibility/adjustability in the form of adjustable energy output, adjustable wavelengths, priority zones, timers, and the like. The sensors of the sensor systems enable the subject embodiments to have the ability to evaluate the treatment area and plan a smart treatment, utilizing more or less energy on the priority zones. The subject embodiments can also be smart from the standpoint of body treatment area such as knee or back, and of skin type, age, overall severity of problems and have the ability to customize the treatment accordingly.

In yet another exemplary embodiment, the lamps are embedded in a flexible sheet of formable material and are integrally molded as strips within a material sheet.

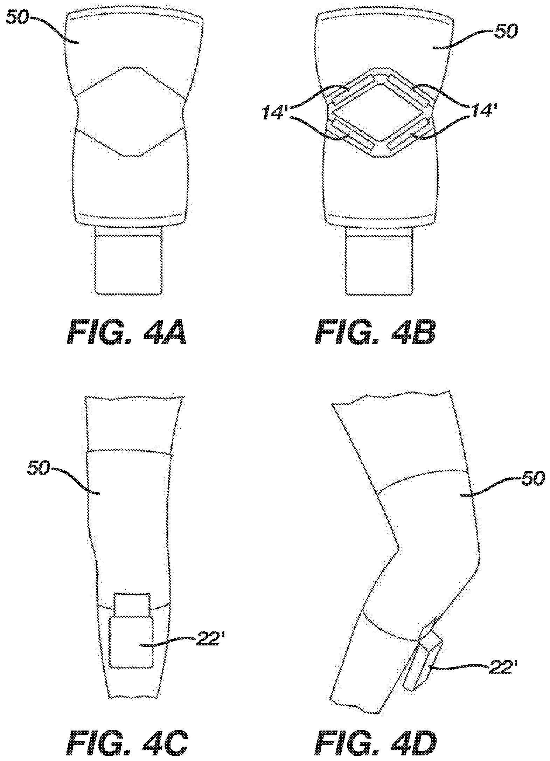

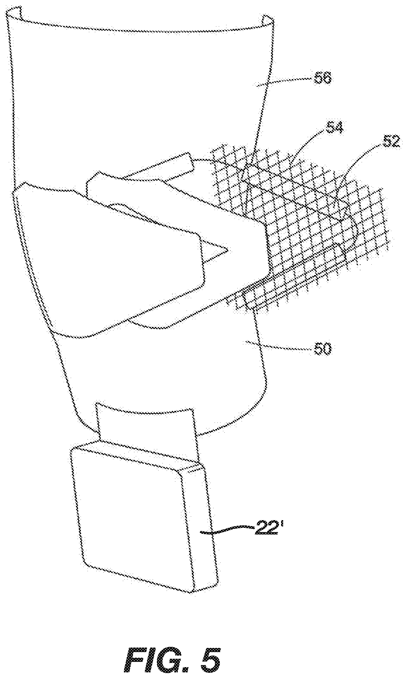

With reference to FIGS. 4A, 4B, 4C, 4D and 5, an exemplary LED bandage is shown where LED strips are arranged in a diamond pattern and the elastic bandage is formed as a unitary sleeve which is pulled over the leg to the knee area. The multi-structural layer of the brace is shown in FIG. 5 and includes an elastic bandage platform 50, a first layer reference material that may be constructed of emergency blanket material 52, LED light strips 54, and a surface layer 56 to cover the strips 54.

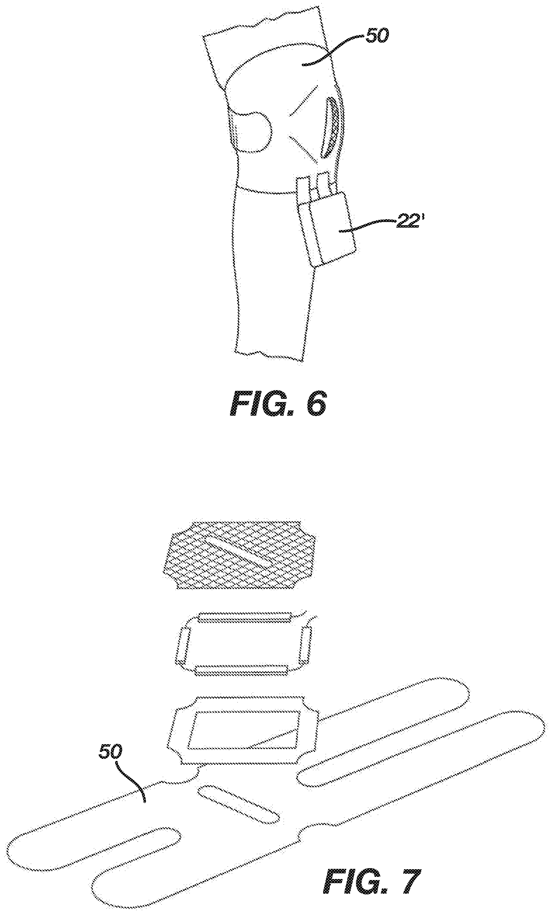

With reference to FIGS. 6 and 7, another alternative embodiment of a knee brace is shown where an elastic bandage is wrapped around a knee as shown in FIG. 6, again the elastic bandage includes a diamond pattern about the patient's kneecap including the multi-layer structures such as is shown in FIG. 7.

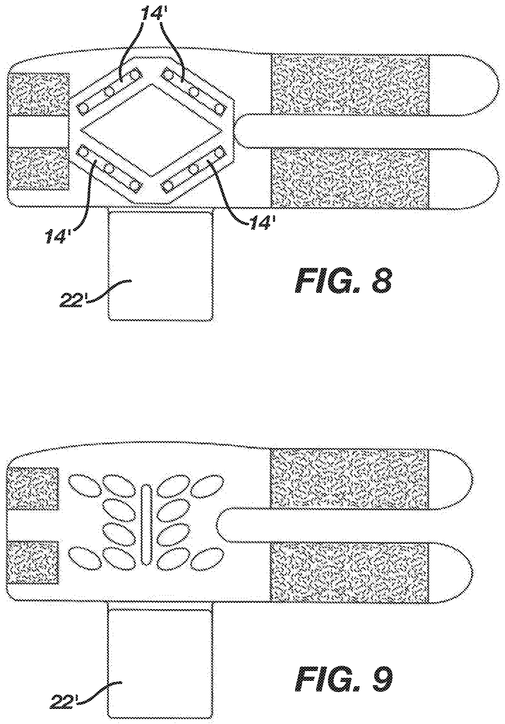

FIGS. 8 and 9 show yet other embodiments which can also function as a wraparound knee brace including the same multi-layer structures such as is shown in FIG. 9.

In other embodiments, the LED strip pattern can be arranged in different placements as shown in the figures to better match treatment to the desired patient treatment area. For example, rather than being equally spaced, the strips can be bunched together in a group, or several groups, where the bandage material is constructed of a material that allows the LED strips to be selectively moved and then affixed to the material at different locations, for example, hook-and-loop fastening fabric.

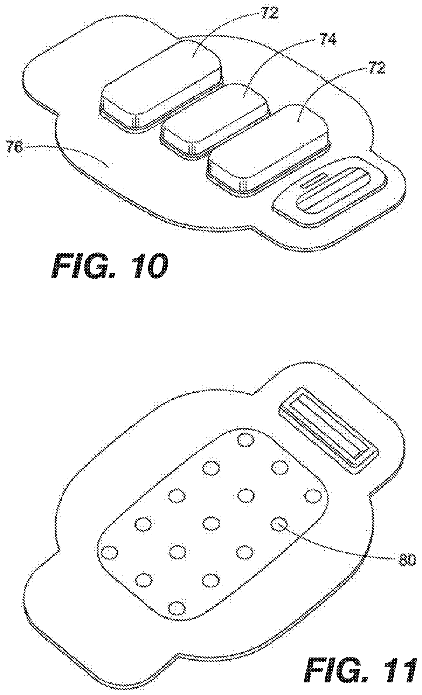

FIGS. 10 and 11 show another embodiment wherein battery energy sources 70 are encased in battery shrouds 72 and operatively connected to a controller 74 attached to a primary fabric layer 76. FIG. 10 shows the top layer of the device away from a user treatment area (not shown). FIG. 11 shows the bottom surface of the device of FIG. 10 wherein the therapeutical lamps of radiation communicate to the treatment area through a plurality of spacer window openings 80.

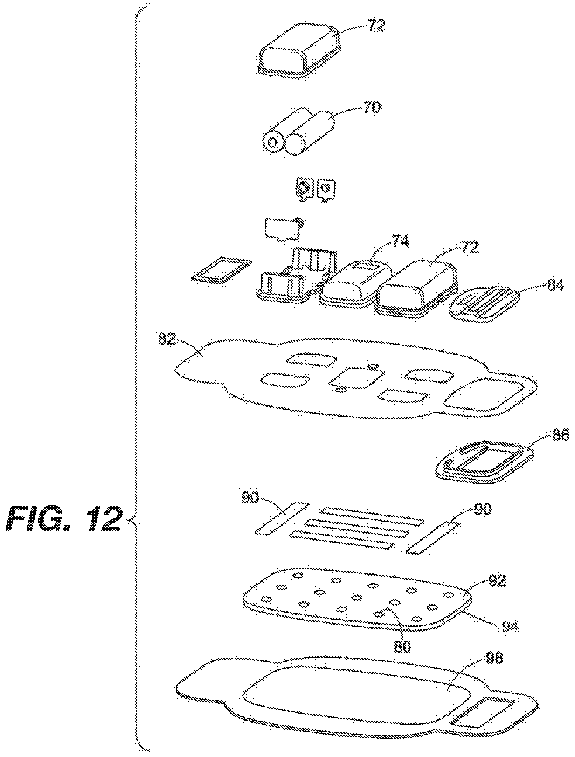

FIG. 12 shows more clearly the component elements of the device. The battery pack 72 and controller 74 are either mechanically attached or heat bonded to the primary fabric layer 82 which can be secured to a patient treatment area through a strap (not shown) received in a buckle 84 and buckle receiver 86 assembly. According to an exemplary embodiment, the therapeutic lamps include a plurality of LED strips 90 mounted on a foam and reflective layer 92 in a manner so that the LEDs are aligned with the windows 80. Power to the LED strips 90 is communicated from the battery 72 via wires (not shown). The foam and reflective layer 92 includes a heat insulator and spacer so that the LEDs mounted on the strips 90 are recessed relative to the opposite surface of the layer 92, rather than the surface on which they are mounted. The strips 90 and layer 92 form a subassembly that in one embodiment is selectively removable and replaceable from and to the device. Layer 92 is highly flexible as are the strips 90 so that the strip 90 and layer 92 subassembly is flexible along a plurality of directions aligned with the areas intermediate the strips for the overall purpose of providing a device which is conformable to properly and comfortably cover a non-flat treatment area. The layer 92 is dimensioned so that the lamps on the LED strips 90 don't break the surface plane of layer 92 on which a reflective layer 94 is attached. According to an exemplary embodiment, reflective layer 94 includes a flexible foil suitable for reflecting the radiant energy of the lamps. A secondary fabric layer covers the foam and reflective layer 92 with a sheer mesh 98 which allows lamp radiation to be communicated to the treatment area with minimal obstruction. The effect is that of a plurality of expanding cones of radiant energy from the lamps of the LED strips 90 that is communicated through the foam layer 92 for therapeutic treatment of the treatment area.

The controller 74 is configured to communicate operational aspects of the device to the user in several ways. When the user actuates an ON switch, an indicator such as a light or beep sounder lets the user know that the device is operating. The controller times the operation to a predetermined limit such as 10 or 15 minutes. In addition, the controller counts usage or cycle sessions to indicate to the user via a controller display, the number of sessions that have been provided by the device and additionally, to disable the device after the LED efficiency in generating therapeutic radiation has been diminished from prior sessions such that the device should no longer be used. The controller also deactivates the indicator light after the session duration has been timed out or may alternatively send another sound beep to the user. Alternatively, the indicator can also provide for indicating battery life or lamp failure.

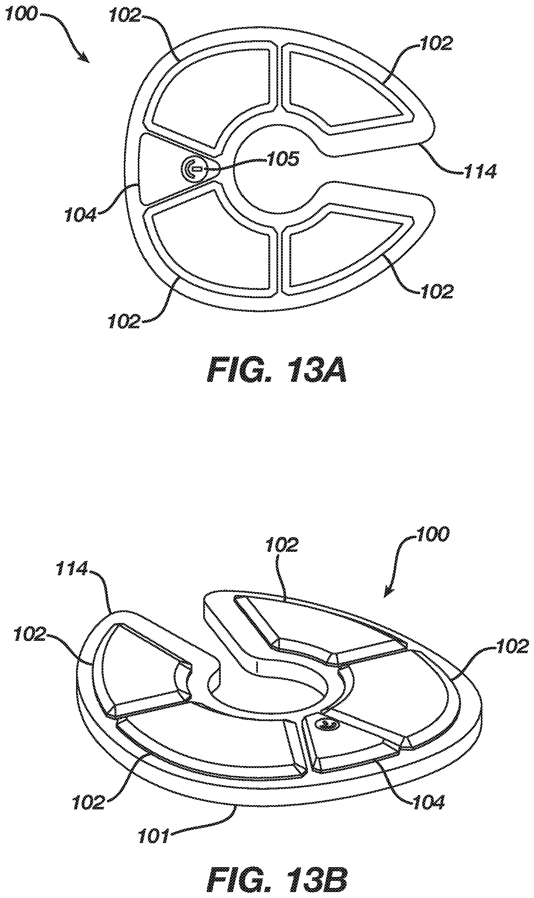

With reference to FIGS. 13-23, illustrated is a stretchable, flexible, and wearable therapeutic lamp platform according to another exemplary embodiment of this disclosure, also referred to as a phototherapy device throughout this disclosure.

FIG. 13A is a top view of the phototherapy device and FIG. 13B is a perspective view of the phototherapy device, as seen by a user. As shown, the phototherapy device is substantially U-shaped or horseshoe shaped, which provides a significant degree of conformability of the device to a plurality of user treatment areas, including but not limited to, ankles, elbows, knees, shoulders, and other body joints, as well as feet. While the application of the phototherapy therapy device shown in FIGS. 13-23 is not limited to any specific user treatment areas, it is especially suited for joints where the device goes around bony joints and treats the muscles/tendons/tissues around the joint.

While the exemplary embodiment described with reference to FIGS. 13-23 includes a U-shaped phototherapy device, it is to be understood other shapes are within the scope of the disclosure, for example, but not limited to, circular shaped, square shaped, rectangular shaped, oval shaped, etc.

As shown in FIGS. 13A and 13B, the exemplary phototherapy device 100 includes a plurality of pods 102 which house one or more batteries and a pod 104 which houses a controller and ON/OFF button switch 105. During operation, the phototherapy device emits therapeutic radiation 101 to a user treatment area to relieve pain and/or provide therapeutic treatment for healing of the user treatment area.

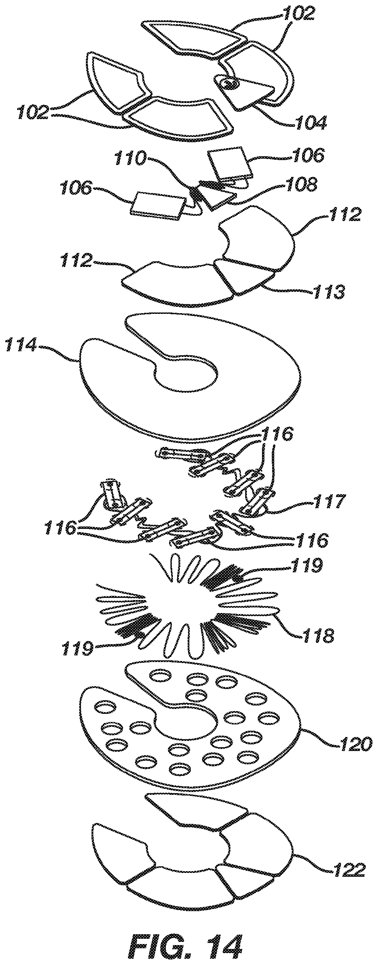

With reference to FIG. 14, illustrated is an exploded view of the stretchable, flexible, and wearable therapeutic lamp platform shown in FIGS. 13A and 13B. The phototherapy device includes hard surface pods 102, a controller pod 140, batteries 106, a controller 108, wires 110 which operatively connect the controller 108 to the batteries 106, pod bottoms 112, controller pod bottom 113, a stretchable and flexible top layer 114, LED strips 116 operatively connected with flexible LED connection wires 117, heating component 118 including temperature sensors 119, a stretchable and flexible bottom layer 120, a reflective layer 122 (note: LED clearance holes not shown) and a biomedical sticky gel.

As shown, the heating element 118 is arranged in a pattern which covers the general shape of the phototherapy device and provides heat to a user treatment area. Essentially a wire, such as a Nichrome® wire driven by the controller 108 provides heat and temperature sensors 119 provide feedback to the controller 108 to regulate the radiated heat provided to the user treatment area, in addition to ramping up the initial heat provided after the phototherapy device is turned ON, for example ramping quickly to maintain a temperature between 40-45 Celsius.

The structure of the phototherapy device provides a stretchable, flexible and conformable, therapeutic lamp platform which can be applied to a variety of user treatment areas. In other words, the phototherapy device is conformable to user treatment area in three dimensions.

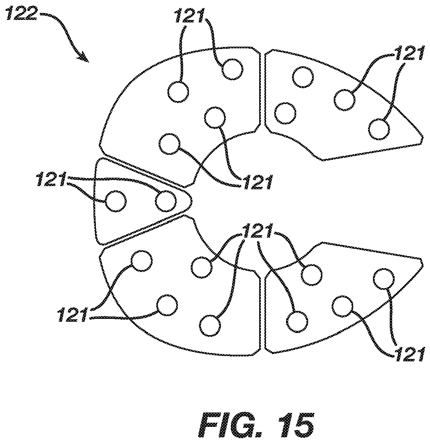

With reference to FIG. 15, illustrated is a plan view of the reflective layer 122 as shown in FIG. 14, except LED clearance holes 121 are shown.

The reflective layer includes a plurality of lamp radiation communication areas 121, such as apertures, clearance holes and/or areas of the reflective layer 122 aligned with the LEDs which are transmissive to the wavelength of the radiation emitted from the LEDs. In other words, the lamp radiation communication areas can be made of a clear or translucent flexible material, where a reflective layer or film, such as a reflective metal foil or PET reflective material is applied to the bottom, i.e. reflective surface of the reflective layer 122, using a masking process to maintain the radiation transmissive characteristics of the radiation communication areas 121. In addition to reflecting lamp radiation, the reflective layer 122 material can include insulating material to contain heat within the user treatment area for effectively treating pain, etc.

As an alternative arrangement, the phototherapy device can integrate the reflective layer 122 with the biomedical sticky gel as a single usable substrate. In other words, the sticky gel, which is replaceable by a user, would include a replaceable reflective layer incorporated into the sticky gel, where a reflective material is encased within the sticky gel.

Sticky gel is a "sticky" adhesive gel compound which removably adheres to the phototherapy device structure reflective layer 122 and a user treatment area. The sticky gel layer is made of a material which also is substantially transparent to the LED lamp radiant emitted by the phototherapy device or includes apertures to communicate the LED lamp radiation. Examples of a suitable material include silicon, hydrogel acrylic and urethane based material.

According to an exemplary embodiment, the sticky gel component includes multiple layers integrated into a single replaceable structure, where a top layer material has properties to provide a bond to the phototherapy device structure and a bottom layer material has properties to provide desirable adhesive properties to a user treatment area. In addition, a third layer material between the top and bottom layers can be provided to act as a structural component to maintain the form of the sticky gel component.

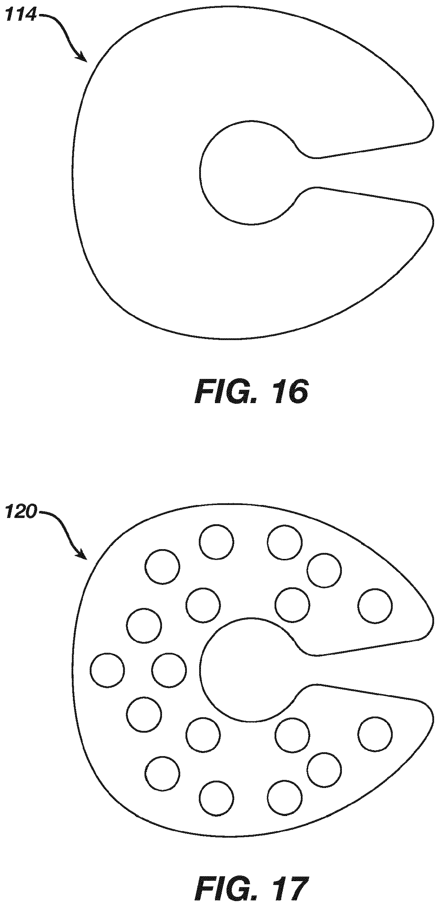

With reference to FIG. 16, illustrated is a plan view of the flexible and stretchable top layer as shown in FIG. 14 and FIG. 17 is a plan view of the flexible and stretchable bottom layer 120 as shown in FIG. 14.

The flexible and stretchable top layer 114 and bottom layer 120 provide a flexible and stretchable housing for LED strips 116, wires 117, an optional heating element 118 and optional temperatures sensors 119. The stretchable layers 114 and 120 can be made from, for example, a low durometer silicone TPE, and/or fabric. As shown in FIG. 17, the flexible bottom layer 120 includes a plurality of LED radiation communication areas, i.e. apertures, to provide radiation to a user treatment area.

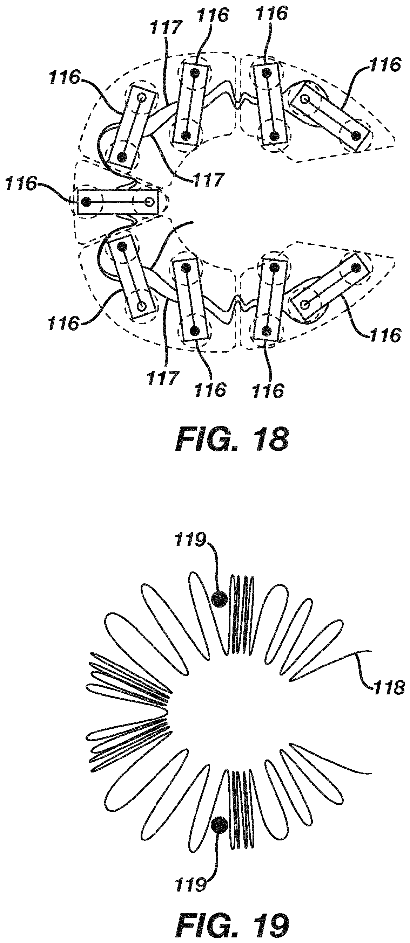

With reference to FIG. 18, shown is a layout of LED strips 116 according to an exemplary embodiment of this disclosure as shown in FIG. 14.

The LED strips 116 include a plurality of LEDs which are operatively connected by wires 117. According to an exemplary embodiment, 18 LEDs of two different wavelengths are provided, where 6 LEDs provide IR (Infrared Spectrum Radiation) for inflammation relief and 12 LEDs provide R (Red Spectrum Radiation).

With reference to FIG. 19, illustrated is an enlarged detail view of the optional heating component 118 and temperature sensor(s) 119 arrangement as shown in FIG. 14.

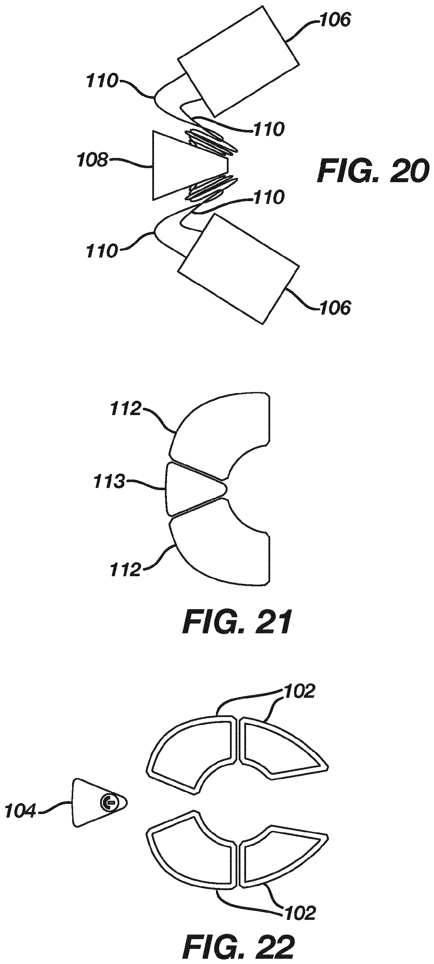

With reference to FIG. 20, illustrated is an enlarged detail view of the circuit board, i.e., controller, and the battery arrangement as shown in FIG. 14.

As shown, included are two batteries 106, a controller 118 and wires 110 which operatively connect batteries 106 to the controller 108. A suitable length of wires 110 provides a stretchable and flexible configuration where the wires 110 are free to expand and contract while maintaining electrical conductivity between the controller 102 and batteries 110, as well as between the controller 108 and LED strips 116.

With reference to FIG. 21, illustrated is an enlarged detail view of the bottom casing for housing the circuit board and batteries as shown in FIG. 14.

With reference to FIG. 22, illustrated is an enlarged detail view of the top casing for housing the circuit board and batteries as shown in FIG. 14.

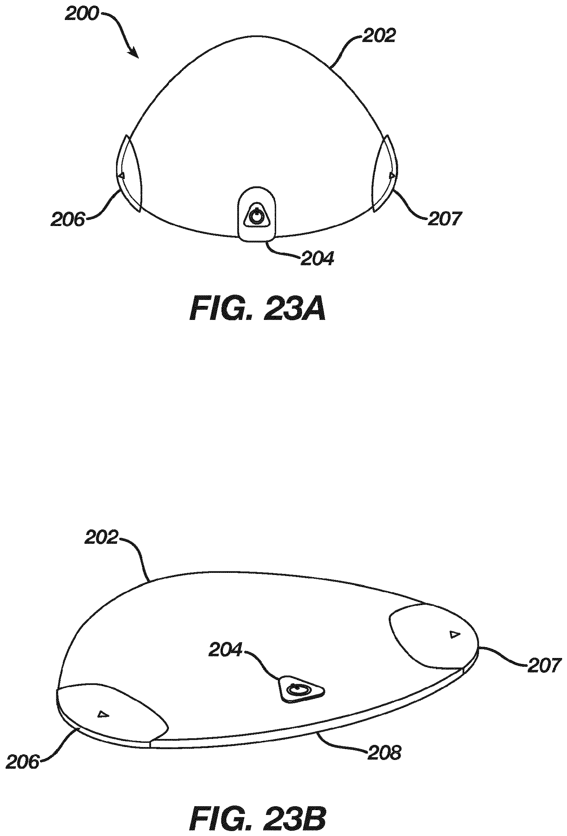

With reference to FIGS. 23-31, illustrated is a flexible wearable therapeutic lamp platform according to another exemplary embodiment of this disclosure, also referred to as a phototherapy device throughout this disclosure. The size and construction of this light therapy platform is especially suited to provide phototherapy to user treatment areas associated with relatively large muscles, such as the lower back, should blades, hip and calves.

With reference to FIG. 23A, illustrated is a top view of the phototherapy device 200 and FIG. 23B is a perspective view of the phototherapy device 200, as seen by a user. As shown, the phototherapy device includes a flexible layer 202, handles 206 and 207 and a control bottom/removable Bluetooth controller housing to operate the phototherapy device. During operation, the phototherapy device 200 emits therapeutic radiation 208 to a user treatment area to relieve pain and/or provide therapeutic treatment for healing of the user treatment area.

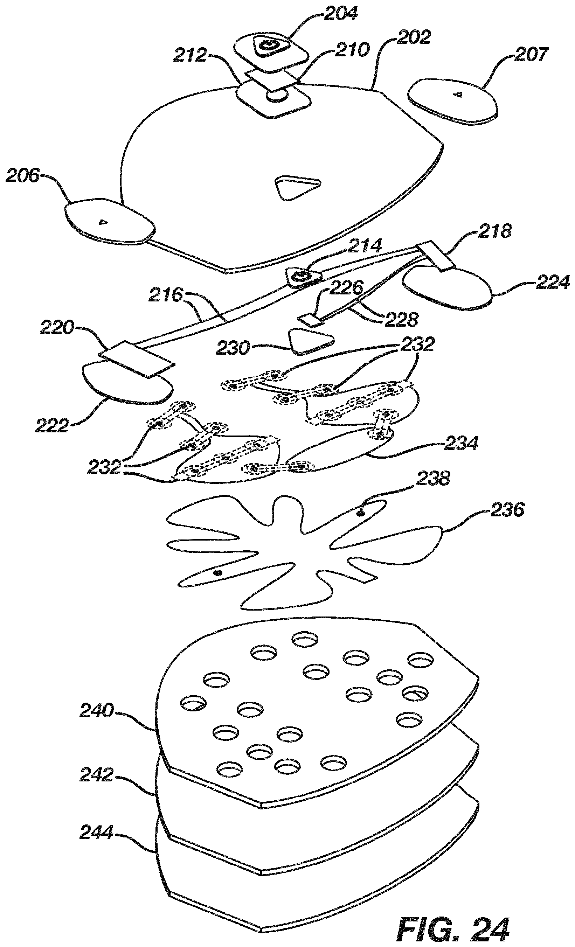

With reference to FIG. 24, illustrated is an exploded view of the flexible wearable phototherapy device shown in FIG. 23. As shown in FIG. 24, the phototherapy device 200 includes a control button/removable Bluetooth controller housing top 204 and bottom 230, a removable Bluetooth controller 210, an intermediate mounting board 212, handle tops 206 and 207, handle bottoms 222 and 224, ON/OFF button switch 214, wires 216 operatively connecting a battery 220 to a controller 218, wires 228 operatively connecting bottom switch 226 to controller 218, LED strips 232 operatively connected with wires 234, a heating component 236 including temperature sensors 238, a bendable/flexible bottom layer 240, a reflective layer 242 (note: LED clearance holes not shown) and a biomedical sticky gel.

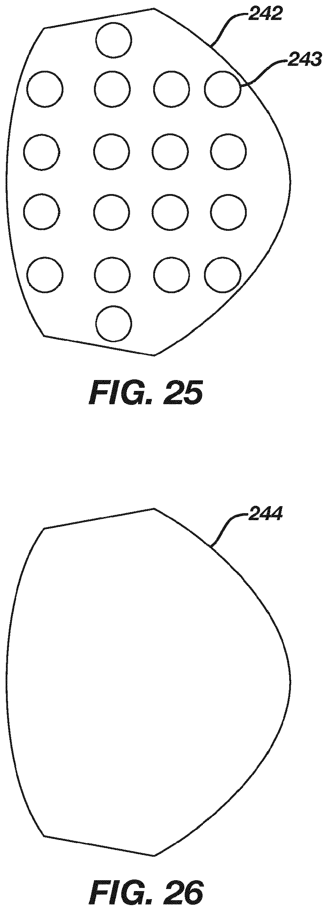

With reference to FIG. 25, illustrated is a plan view of the reflective layer as shown in FIG. 24, with the LED clearance holes 243 shown.

With reference to FIG. 26, illustrated is a plan view of the biocompatible sticky gel layer as shown in FIG. 24.

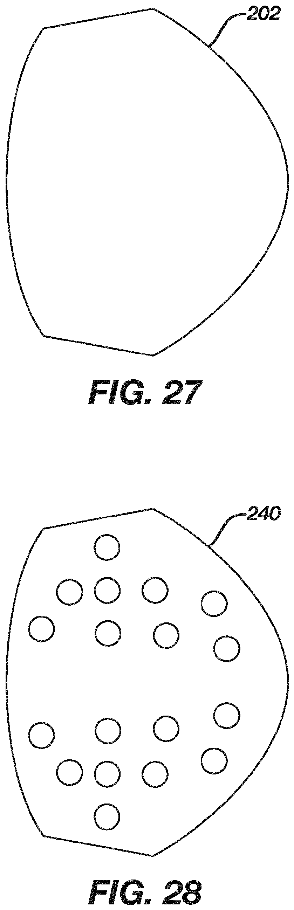

With reference to FIG. 27, illustrated is a plan view of the flexible layer as shown in FIG. 24, and FIG. 28 illustrates a plan view of the bendable bottom layer as shown in FIG. 24. Materials suitable for construction of these layers includes low durometer SAN, Neoprene, TPE, silicon, and fabric.

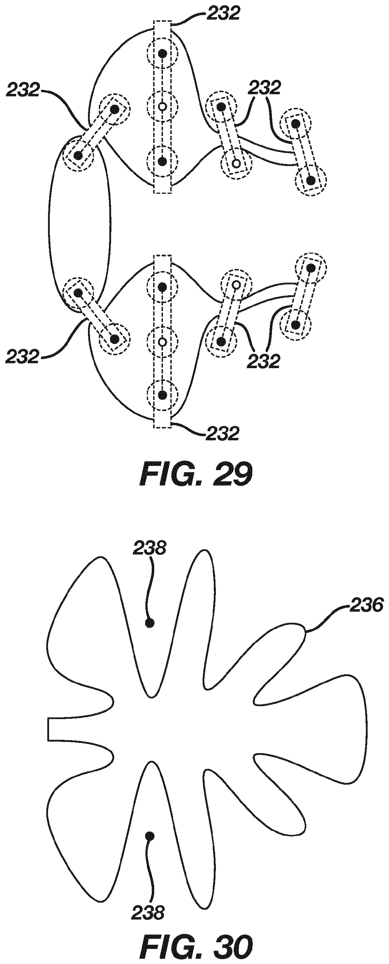

With reference to FIG. 29, shown is a layout of LED strips according to an exemplary embodiment of this disclosure as shown in FIG. 14.

With reference to FIG. 30, illustrated is an enlarged detail view of the optional heating component and temperature sensor(s) arrangement as shown in FIG. 24.

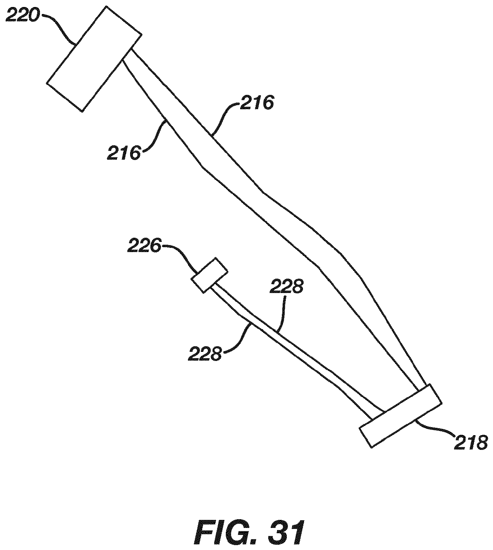

With reference to FIG. 31, illustrated is an enlarged detail view of the circuit board 218, i.e., controller separate power button optional Bluetooth antenna 226, and the battery arrangement 220 as shown in FIG. 24.

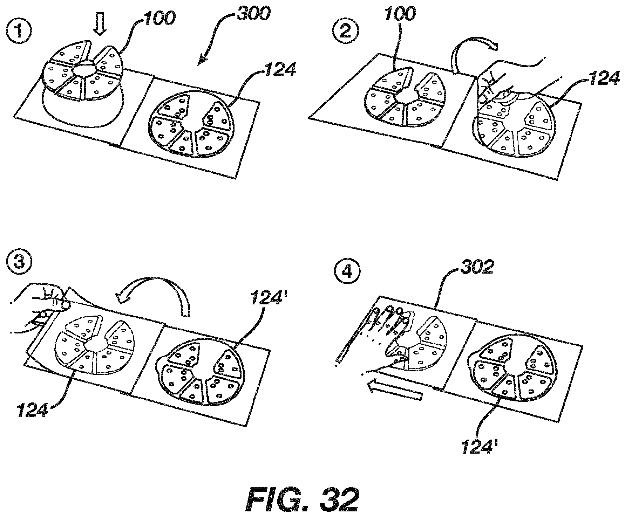

With reference to FIG. 32, illustrated is a four step process to replace the biomedical sticky gel component of a phototherapy device 100 previously described.

After a user removes the used sticky gel component from the phototherapy device, the user initially places the phototherapy device bottom-side-up in the docking area of the carrier 300 shown.

Next, at step 2, the user removes an unused sticky gel component from the carrier 300 and places the unused sticky gel component on the phototherapy device as shown in step 3.

Finally, the user applies pressure to the sticky gel component backing layer 302 to adhere the sticky gel component to the phototherapy device as shown at step 4.

Possible adhesive gel carrier designs include a boot package similar to a foldable travel case, roll type packaging where a user unrolls the package to remove the next adhesive gel, and a pencil case package.

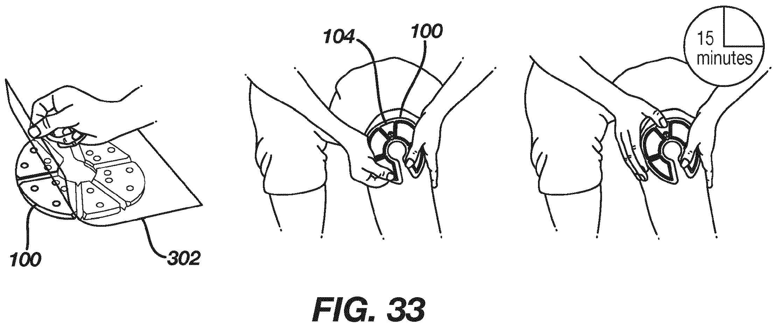

With reference to FIG. 33, shown is a process to apply the phototherapy device including a replaced sticky gel component to a user treatment area. Initially, the user removes the sticky gel replacement backing 302. Next, the user applies, with pressure, the phototherapy device 100 to a treatment area and activates the ON/OFF button switch 105 to begin a phototherapy treatment session, as shown in FIG. 33.

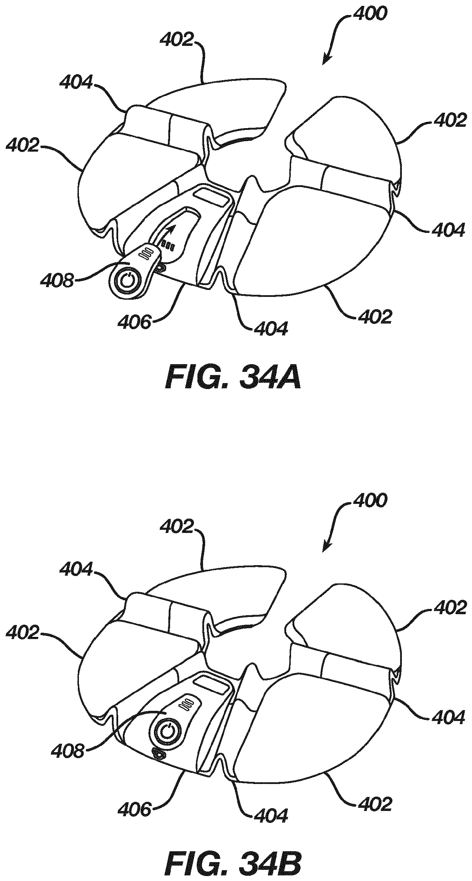

FIGS. 34A and 34B illustrate another stretchable wearable therapeutic lamp platform 400 according to an exemplary embodiment of this disclosure. The therapeutic lamp platform including a SIM (Subscriber Identity Module) operatively connected to the platform.

As shown, the phototherapy device 400 includes a plurality of soft surface pods 402, a plurality of expandable LED wire encasements, a controller 406 and a SIM Card device 408, which is used to activate the phototherapy device to provide a predetermined number of dosages of light therapy treatment, for example, 2-100 or any other number of dosages/sessions, including an unlimited number of treatments.

According to one exemplary embodiment, the SIM Card is a consumable product purchased by a user to provide a limited number of treatments before being required to purchase another SIM Card or electronically purchase the additional dosages for the depleted SIM Card.

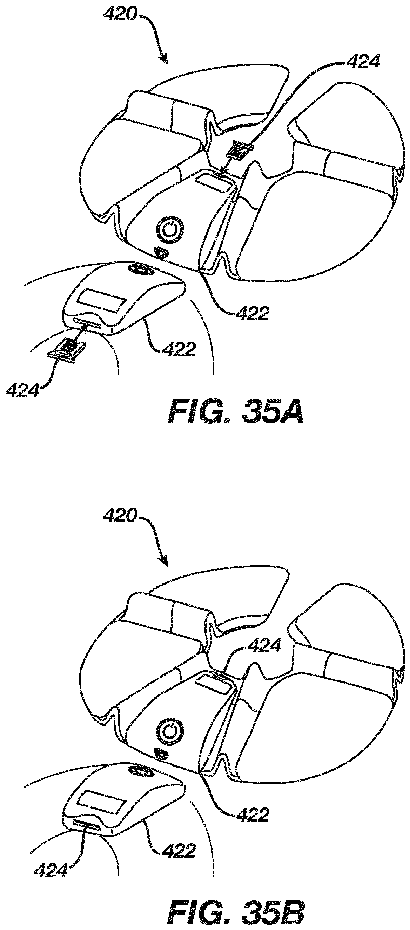

FIGS. 35A and 35B illustrate an exemplary embodiment of a wearable therapeutic lamp platform 420 including a SIM top end card slot 424 and controller 422.

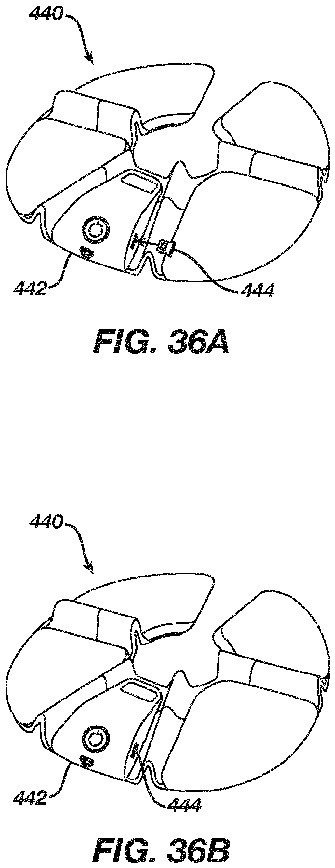

FIGS. 36A and 36B illustrate an exemplary embodiment of a wearable therapeutic lamp platform 440 including a SIM side card slot 444 and associated controller 442.

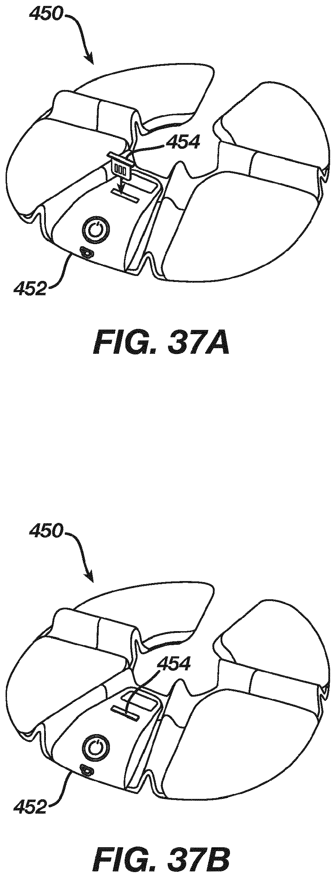

FIGS. 37A and 37B illustrate an exemplary embodiment of a wearable therapeutic lamp platform 450 including a SIM retro video game card slot 454 and associated controller 452.

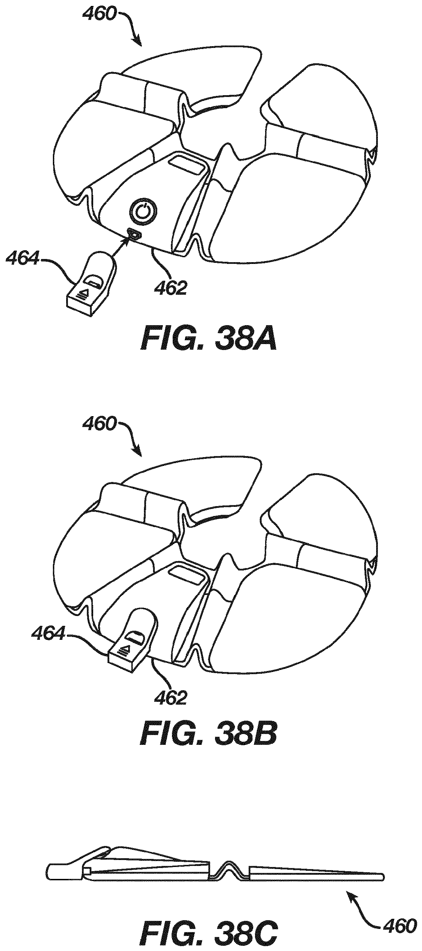

FIGS. 38A, 38B and 38C illustrate an exemplary embodiment of a wearable therapeutic lamp platform 460 including a combination SIM and SIM card reader 464 and associated controller 462.

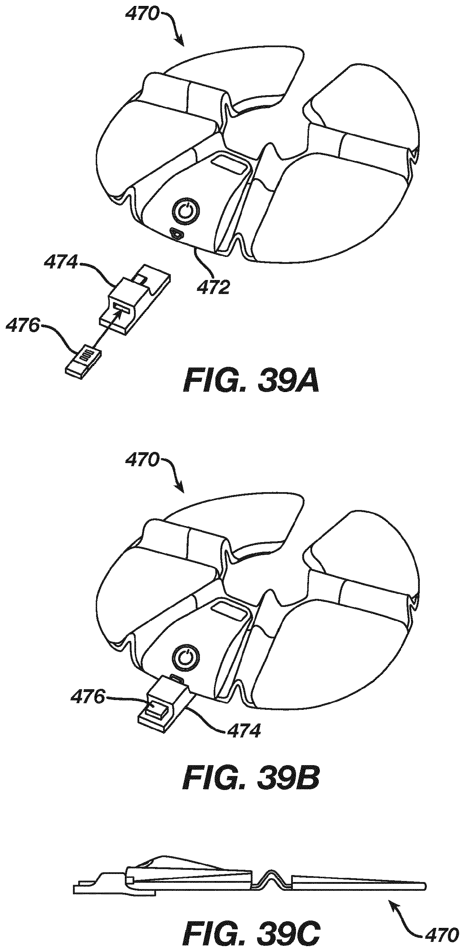

FIGS. 39A, 39B and 39C illustrate an exemplary embodiment of a wearable therapeutic lamp platform 470 including a combination SIM 476 and SIM card reader 474 outside of the main pod, and associated controller 472.

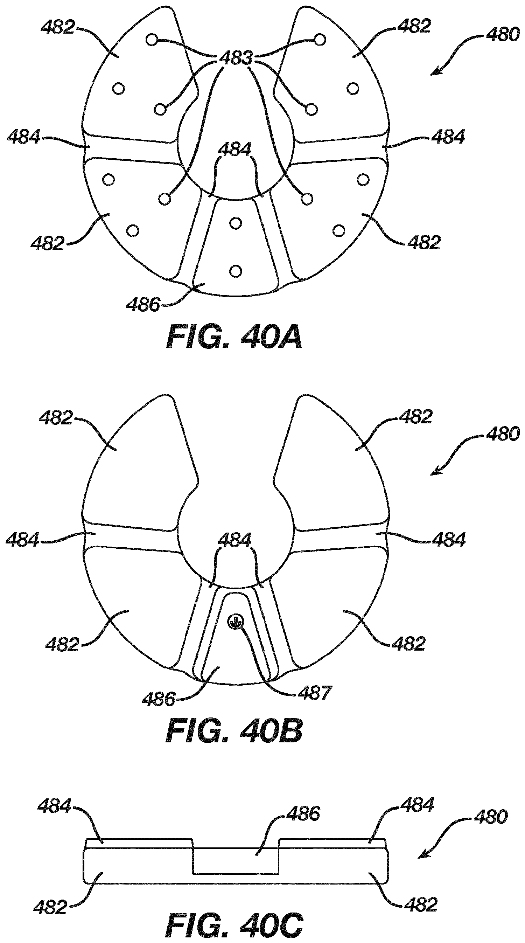

FIGS. 40A, 40B and 40C illustrate a stretchable wearable lamp platform according to an exemplary embodiment of this disclosure.

As shown, the phototherapy device 480 includes a plurality of soft surface pads 482 having disposed on the bottom surface thereof a plurality of apertures 483 operatively associated with the LED lamps and that are operatively interconnected by a plurality of expandable LED wire encasements 484 and a controller 486 having an ON/OFF button switch 487.

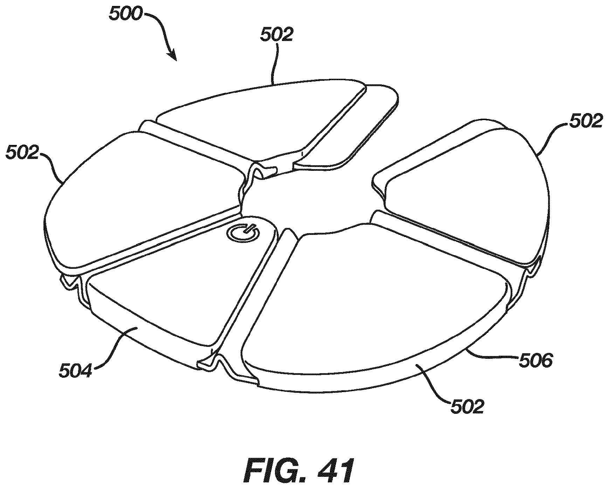

FIG. 41 is a perspective view of an exemplary embodiment of another stretchable wearable lamp platform 500 according to this disclosure.

As shown, the phototherapy device 500 includes a plurality of hard surface pods 502 and a controller pod 504, where the device emits therapeutic lamp radiation 506.

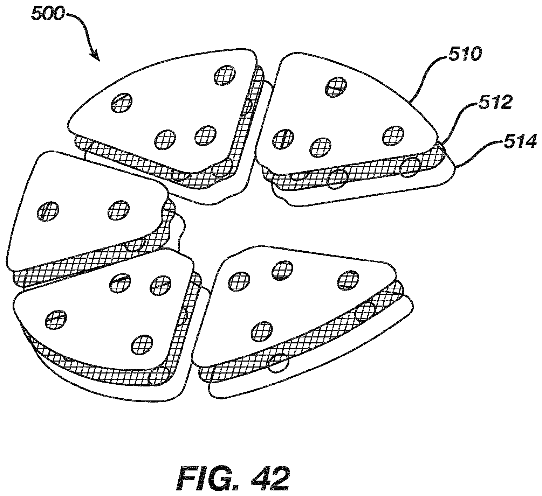

FIG. 42 illustrates the adhesive layer construction 508 of the stretchable wearable lamp platform shown in FIG. 41.

As shown, the adhesive layer construction 508, i.e. sticky gel component, includes an adhesive layer 510 for attaching to the phototherapy device, a mid-layer structural layer 512 and an adhesive layer for the skin of the user treatment area.

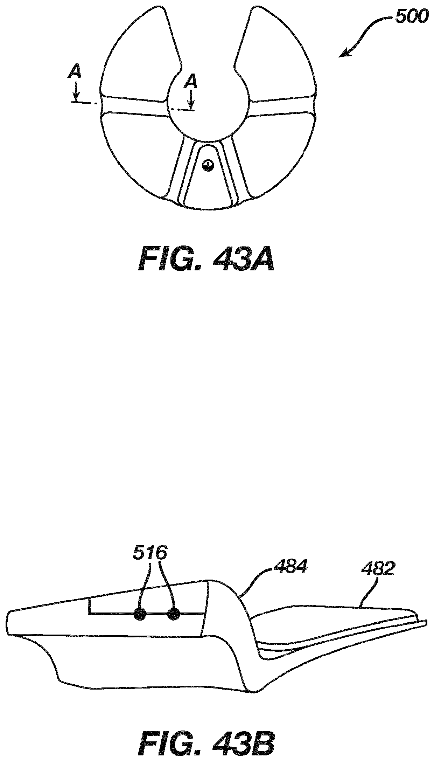

FIGS. 43A and 43B are detail views of the structural and electrical interconnection of the pods associated with the stretchable wearable lamp platform shown in FIG. 41.

As shown, the soft surface pod 482 is operatively connected to an expandable LED wire encasement 484 where the radial configuration provides for flexibility and stretchability of wires 516 which drive the LEDs.

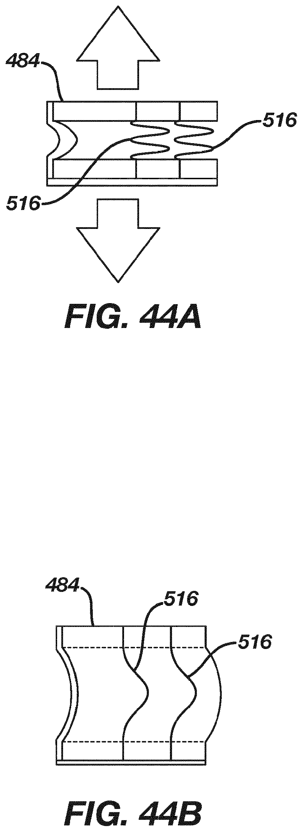

FIGS. 44A and 44B are additional detail views of the structural and electrical interconnections of the pods associated with the stretchable wearable lamp platform shown in FIG. 41, where FIG. 44A shows the wire encasement in a contracted form and FIG 44B shows the wire encasement in an expanded form.

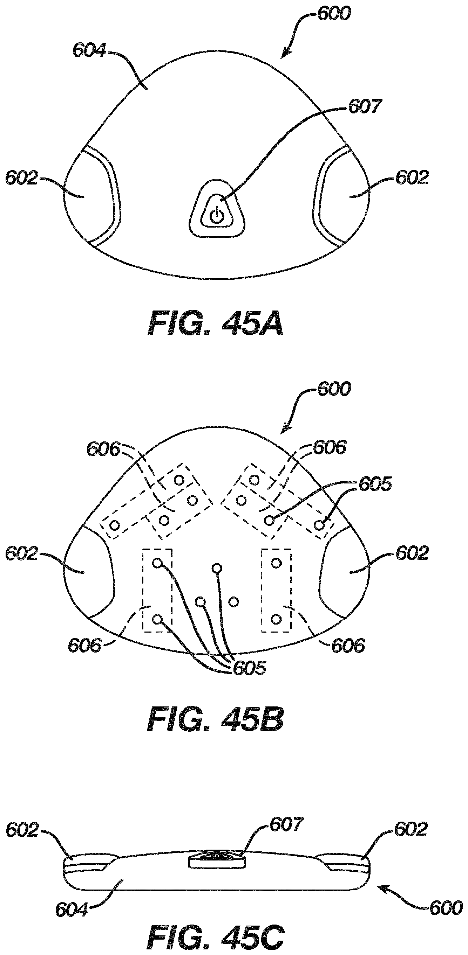

FIGS. 45A, 45B and 45C are images of a flexible wearable lamp platform 600 according to an exemplary embodiment of this disclosure.

As shown, the phototherapy device 600 includes handles 602, a flexible stretchable layer 604, having disposed on the bottom surface thereof a plurality of apertures 605 operatively associated with the LED disposed on LED strips 606 contained therein and an ON/OFF control switch encasement 607.

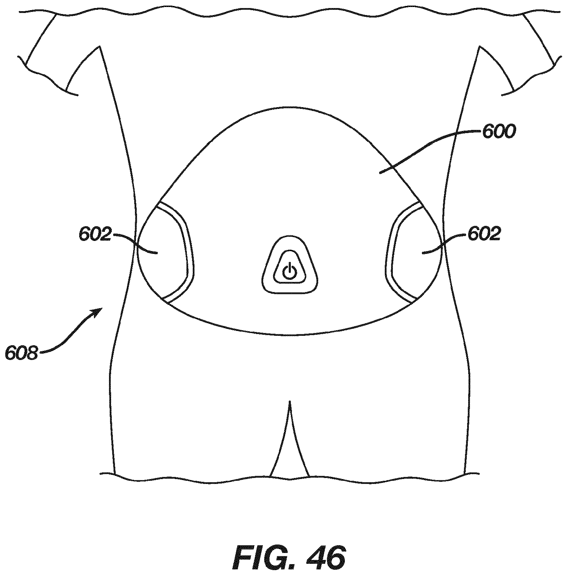

FIG. 46 is an image of the flexible wearable lamp platform shown in FIGS. 45A, 45B and 45C, applied to a user's lower back area .

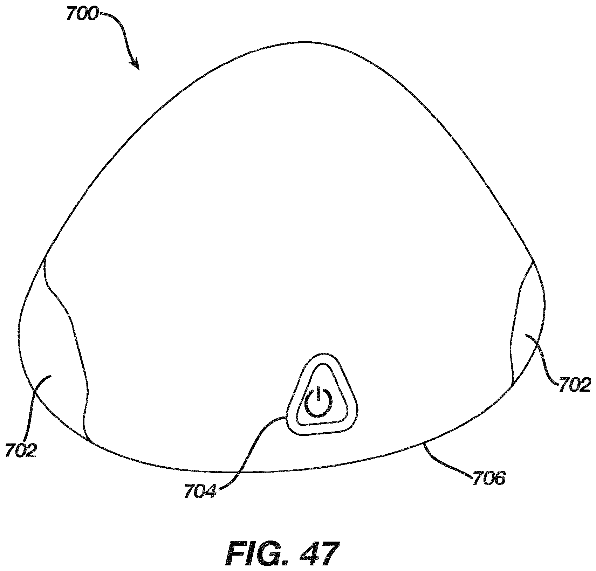

FIG. 47 illustrates another flexible wearable lamp platform 700 according to an exemplary embodiment of this disclosure, the platform 700 including handles 702, and an on/off control encasement, where the device provides therapeutic lamp radiation 706.

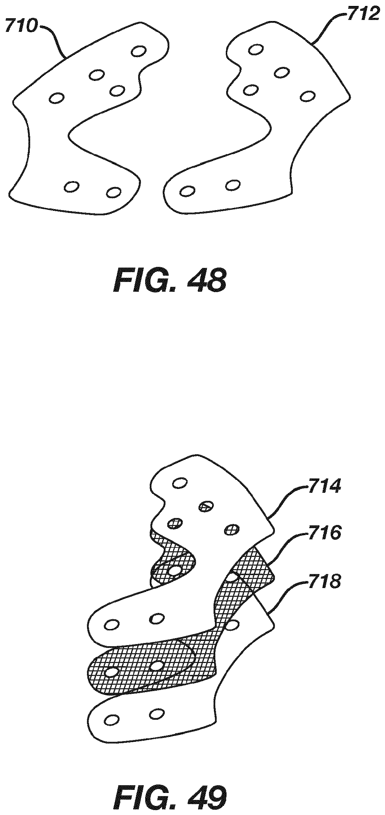

FIG. 48 illustrates an exemplary embodiment of the replaceable adhesive layers included in the flexible wearable lamp platform shown in FIG. 46, and FIG. 49 illustrates the layered construction of the replaceable adhesive layers shown in FIG. 48.

As shown, included is a first adhesive pad portion 710 and a second adhesive pad portion 712. Adhesive pads 710 and 712 include an adhesive layer to attach to the phototherapy device structure, a mid-layer structural component 716 and an adhesive layer 718 for attaching to the skin.

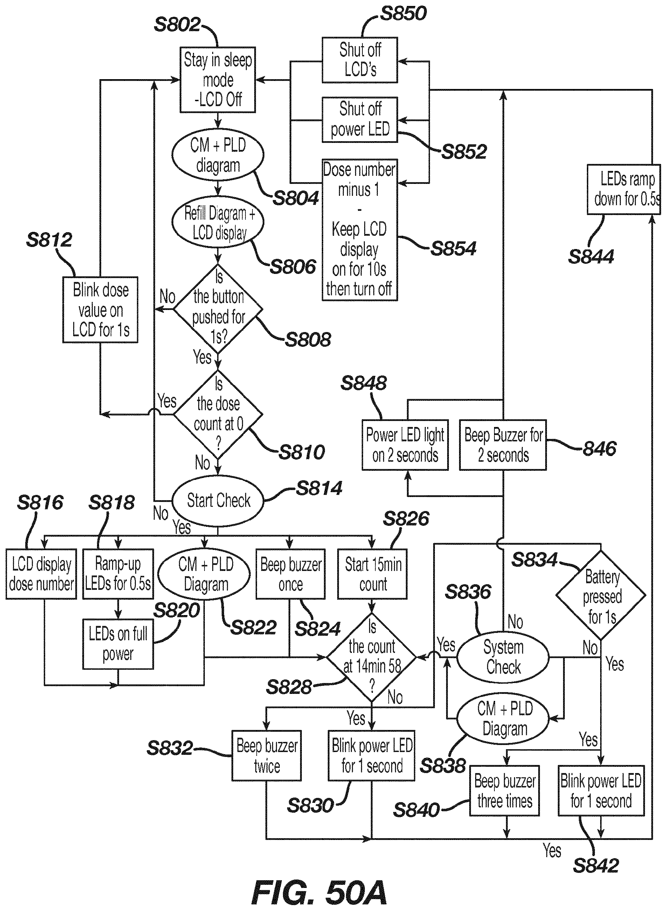

With reference to FIGS. 50A-50E, shown are flow charts of a control program to operate a flexible wearable therapeutic lamp platform according to an exemplary embodiment of this disclosure.

FIG. 50A is a flow chart of the main operational control program, which operates as follows:
Initially at step S802, the phototherapy device is under power and operates in a Sleep Mode with the LCD display off.

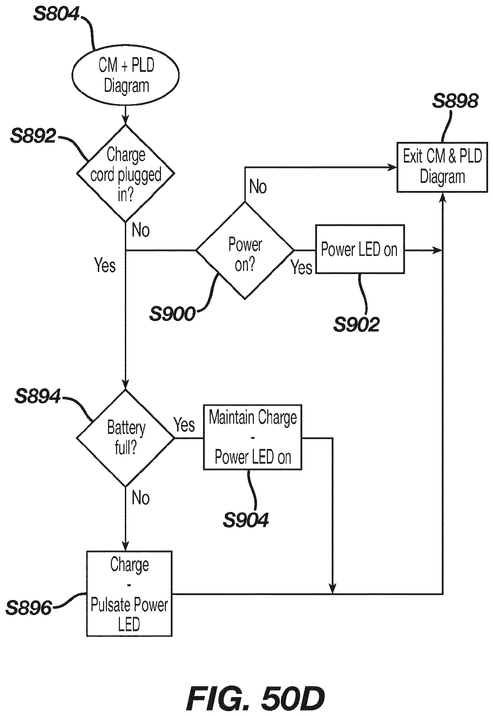

During Sleep Mode, the control program executes a CM & PLD (Charge Manager and Power LED Display) subcontrol program S804, as shown in FIG. 50D.

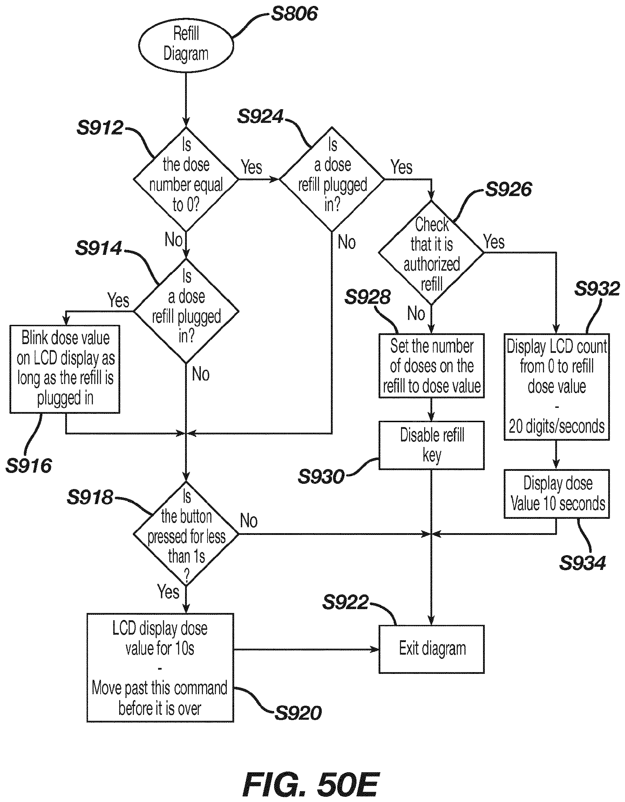

Next, the control program executes a Refill and LCD Display subcontrol program S806, as shown in FIG. 50E.

Next, at step S808, the control program monitors the control ON/OFF button to determine if the ON/OFF button is pushed for 1 second. If not, the control program returns to step S802. If yes, the control program next executes step S810 to determine if the device has remaining dosages available.

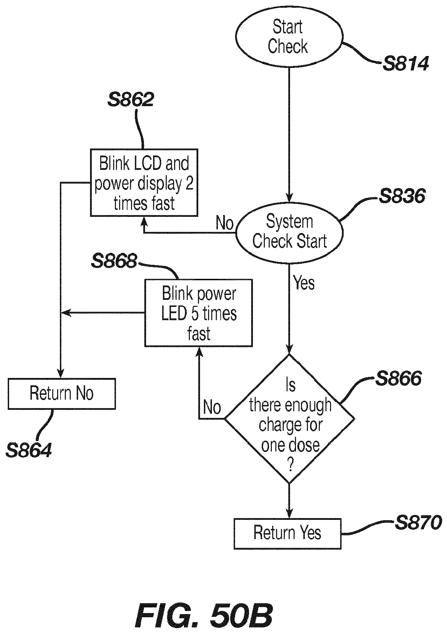

If the dosage counter is zero, then the control program executes step S812 and blinks the dose value of "0" on the LCD to notify the user that no dosages are available. If there are remaining dosages, the control program executes step S814 to perform a Start Check subcontrol as shown in FIG. 50B.

If the Start Check subcontrol program is not executed satisfactorily, the control program returns to step S802.

After the Start Check subcontrol program is executed successfully, the control program executes step S816 to display the dose number, step S818 and S820 to ramp-up the LED to full power in 0.5 seconds, executes CM & PLD subcontrol program S822, step S824 to beep the buzzer once, and step S826 to start a 15 minute dosage session counter.

Next, at step S828, the control program monitors the dosage session counter until the active dosage session is completed, at which time step S832 beeps the buzzer twice and, at step S830, LED power is blinked for 1 second, both steps S832 and S830 notifying the user the currently active dosage session has been completed.

Next, the control program executes step S844 to ramp down LED power in 0.5 seconds, then the control program executes step S850 to shut off the LEDs, step S852 to shut off power to the LEDs, and step S854 to display available number of doses to the user, which is one less than previously available and displayed at step S816.

Next, the control program reviews Sleep Mode at step S802.

If, at step S828, the control program has not yet reached the end of the current active dosage session, the control program executes step 834 to monitor the ON/OFF button state, where, if the ON/OFF button is not pressed for 1 second, the control program executes subcontrol program System Check at step S836 and subcontrol program CM & PLD control program at step S838 until the current active dosage session is completed.

In the event the user presses the ON/OFF button for 1 second during the dosage session, the control program terminates the current active dosage session by executing step S840 to beep the buzzer three times and step S842 to blink the LED power for 1 second, and then executes steps S844, S850, S852 and S854 as previously described.

FIG. 50B is a flow chart of the Start Check subcontrol program S814.

Initially, at step S836, the system Check Start subcontrol program S836 is executed and if not completed successfully, the subcontrol program performs step S862 to blink the LCD display and LED power to notify user at the failure, and returns to main program at step S864 indicating "NO" passage of Start Check.

After the successful completion of step S836, the subcontrol program executes step S866 to determine if there is enough battery power/charge to complete a phototherapy dose; if there is not, the subcontrol program blinks the LEDs 5 times fast to notify the user and returns to the main program at step S864.

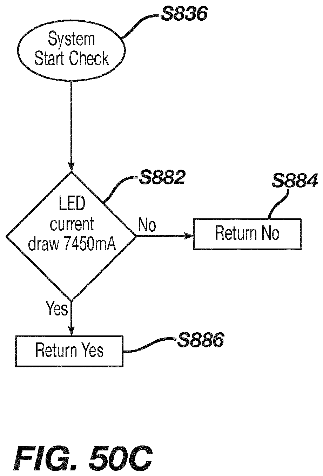

FIG. 50C is a flow chart of the system Check Start subcontrol program, S836.

The system Check Start subcontrol program monitors LED power draw at step S882 and after the LED power is greater than 450 mA, indicating a proper dosage radiant energy amount, the subcontrol program returns to the main program at S886 to continue executing the main control program to provide a dosage, otherwise a "NO" is returned at step S884 until adequate power is drawn by the LEDs.

FIG. 50D is a flow chart of the CM & PLD subcontrol program.

Initially, the subcontrol program S804 determines if the charge cord is plugged in at step S892. If the charge cord is not plugged in, step S900 is executed to determine if the power is ON, and, if it is, the LEDs are powered at step S902, and, at step S898, the CM & PLD subcontrol program is exited. If the power is determined to be OFF at step S900, the CM & PLD subcontrol program is exited at step S898.

If the charge cord is determined to be plugged in at S892, the subcontrol program determines at step S894 if the battery is fully charged. If YES, step S904 maintains charge and the power ON LED is illuminated and step S898 is performed to exit the CM & PLD subcontrol program. If the battery is determined to require charging at step S894, the subcontrol program executes step S896 to charge the battery while pulsating the power LED until the device is fully charged.

After completion of the battery charging step S896, the subcontrol program performs step S898 to exit the CM & PLD subcontrol program.

FIG. 50E is a flow chart of the Refill and LED display subcontrol program S806.

Initially, the subcontrol program determines if the dosage number is equal to 0. If it is equal, step S924 is executed to determine if a refill cartridge is plugged in the device. If no refill cartridge is available, the subcontrol program executes step S916 to blink the dose value on the display to notify the user a refill is required.

After step S924 determines a refill cartridge is available, step S926 determines if the refill cartridge is authorized. If the refill cartridge is not authorized, steps S928 and S930 are executed to disable the refill cartridge. If the refill cartridge is determined to be authorized, the subcontrol program executes step S923 to display the addition of the refill doses amount and step S934 displays the total number of available doses for 10 seconds to the user.

At step S918, the subcontrol program monitors the ON/OFF button and if the ON/OFF button is not pressed, the subcontrol program exits at step S922. If the ON/OFF button is pressed for 1 second, step S920 is executed to display on the LCD the dose value for 10 seconds and then performs step S922 to exit the subcontrol program.

Some portions of the detailed description herein are presented in terms of algorithms and symbolic representations of operations on data bits performed by conventional computer components, including a central processing unit (CPU), memory storage devices for the CPU, and connected display devices. These algorithmic descriptions and representations are the means used by those skilled in the data processing arts to most effectively convey the substance of their work to others skilled in the art. An algorithm is generally perceived as a self-consistent sequence of steps leading to a desired result. The steps are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

It should be understood, however, that all of these and similar terms are to be associated with the appropriate physical quantities and are merely convenient labels applied to these quantities. Unless specifically stated otherwise, as apparent from the discussion herein, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The exemplary embodiment also relates to an apparatus for performing the operations discussed herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, and magnetic-optical disks, read-only memories (ROMs), random access memories (RAMs), EPROMs, EEPROMs, magnetic or optical cards, or any type of media suitable for storing electronic instructions, and each coupled to a computer system bus.

The algorithms and displays presented herein are not inherently related to any particular computer or other apparatus. Various general-purpose systems may be used with programs in accordance with the teachings herein, or it may prove convenient to construct more specialized apparatus to perform the methods described herein. The structure for a variety of these systems is apparent from the description above. In addition, the exemplary embodiment is not described with reference to any particular programming language. It will be appreciated that a variety of programming languages may be used to implement the teachings of the exemplary embodiment as described herein.

A machine-readable medium includes any mechanism for storing or transmitting information in a form readable by a machine (e.g., a computer). For instance, a machine-readable medium includes read only memory ("ROM"); random access memory ("RAM"); magnetic disk storage media; optical storage media; flash memory devices; and electrical, optical, acoustical or other form of propagated signals (e.g., carrier waves, infrared signals, digital signals, etc.), just to mention a few examples.

The methods illustrated throughout the specification, may be implemented in a computer program product that may be executed on a computer. The computer program product may comprise a non-transitory computer-readable recording medium on which a control program is recorded, such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other tangible medium from which a computer can read and use.

Alternatively, the method may be implemented in transitory media, such as a transmittable carrier wave in which the control program is embodied as a data signal using transmission media, such as acoustic or light waves, such as those generated during radio wave and infrared data communications, and the like.

It will be appreciated that variants of the above-disclosed and other features and functions, or alternatives thereof, may be combined into many other different systems or applications. Various presently unforeseen or unanticipated alternatives, modifications, variations or improvements therein may be subsequently made by those skilled in the art which are also intended to be encompassed by the following claims.

## Claims

1. A stretchable and flexible wearable phototherapy device (100) comprising:
a plurality of radiant energy pods (102), which house one or more integrated and wearable batteries (106) each pod including one or more radiant lamps to provide radiant energy to a user treatment area, and each pod stretchably and flexibly connected to one or more other pods; and
a control pod (104) which houses a controller and ON/OFF button switch (105) and is stretchably and flexibly connected to one or more radiant energy sections, the control pod operatively connected to the radiant energy pods and configured to control an operation of the radiant lamps,
wherein the pods are stretchably and flexibly connected such that they form a horseshoe shape.

2. The stretchable and flexible wearable device according to claim 1, the control pod (104) including a power source to operatively power the one or more radiant lamps.

3. The stretchable and flexible wearable phototherapy device according to claim 1 or claim 2, further comprising:
a stretchable and flexible reflective wall (122) including a plurality of radiant energy communication areas (483) aligned with the radiant lamps and disposed to communicate the radiant energy to the user treatment area.

4. The stretchable and flexible wearable phototherapy device according to any preceding claim, further comprising:
a stretchable and flexible adhesive layer (508) including a first subsurface (510) and a second subsurface (514), the first subsurface removably attached to the wearable phototherapy device and the second subsurface operatively associated with removably attaching the wearable phototherapy device to the user treatment area.

5. The stretchable and flexible wearable device according to any preceding claim, wherein the plurality of radiant lamps include one or both of Red and Infrared wavelength radiant energy.

6. The stretchable and flexible wearable device according to any preceding claim, further comprising an integrated and wearable microcontroller (108) configured to control an operation of the phototherapy device.

7. The stretchable and flexible wearable device according to any preceding claim, wherein one or more of the reflective wall (122) and adhesive layer (501) is a silicon and/or urethane based material.

## Patentansprüche

1. Dehnbare und flexible tragbare Phototherapievorrichtung (100), die umfasst:
mehrere Strahlungsenergiehülsen (102), die eine oder mehrere integrierte und tragbare Batterien (106) aufnehmen, wobei jede Hülse eine oder mehrere Strahlungslampen beinhaltet, die Strahlungsenergie zu einem Behandlungsbereich eines Benutzers bereitstellen, und wobei jede Hülse dehnbar und flexibel mit einer oder mehreren anderen Hülsen verbunden ist; und
eine Steuerungshülse (104), die eine Steuerung und einen EIN-/AUS-Tastenschalter (105) aufnimmt und dehnbar und flexibel mit einem oder mehreren Strahlungsenergiebereichen verbunden ist, wobei die Steuerungshülse mit den Strahlungsenergiehülsen wirkverbunden und ausgebildet ist, einen Betrieb der Strahlungslampen zu steuern,
wobei die Hülsen derart dehnbar und flexibel verbunden sind, dass sie eine Hufeisenform ausbilden.

2. Dehnbare und flexible tragbare Vorrichtung nach Anspruch 1, wobei die Steuerungshülse (104) eine Leistungsquelle beinhaltet, um die eine oder mehreren Strahlungslampen im Betrieb mit Leistung zu versorgen.

3. Dehnbare und flexible tragbare Phototherapievorrichtung nach Anspruch 1 oder Anspruch 2, ferner umfassend:
eine dehnbare und flexible reflektierende Wand (122) mit mehreren Strahlungsenergieübertragungsbereichen (483), die mit den Strahlungslampen ausgerichtet und angeordnet sind, die Strahlungsenergie zu dem Behandlungsbereich des Benutzers zu übertragen.

4. Dehnbare und flexible tragbare Phototherapievorrichtung nach einem der vorhergehenden Ansprüche, ferner umfassend:
eine dehnbare und flexible Klebeschicht (508) mit einer ersten Teilfläche (510) und einer zweiten Teilfläche (514), wobei die erste Teilfläche entfernbar an der tragbaren Phototherapievorrichtung angebracht ist und die zweite Teilfläche im Betrieb mit einem entfernbaren Anbringen der tragbaren Phototherapievorrichtung an dem Behandlungsbereich des Benutzers in Zusammenhang steht.

5. Dehnbare und flexible tragbare Vorrichtung nach einem der vorhergehenden Ansprüche, wobei die mehreren Strahlungslampen eines oder beides von Strahlungsenergie mit Rotlicht- und Infrarotwellenlänge umfasst.

6. Dehnbare und flexible tragbare Vorrichtung nach einem der vorhergehenden Ansprüche, ferner umfassend eine integrierte und tragbare Mikrosteuerung (108), die ausgebildet ist, einen Betrieb der Phototherapievorrichtung zu steuern.

7. Dehnbare und flexible tragbare Vorrichtung nach einem der vorhergehenden Ansprüche, wobei eine oder mehrere der reflektierenden Wand (122) und der Klebeschicht (501) ein Material auf Silizium- oder Urethanbasis ist.

## Revendications

1. Dispositif de photothérapie portable extensible et flexible (100) comprenant :
une pluralité de boîtiers d'énergie rayonnante (102), qui contiennent une ou plusieurs piles intégrées et portables (106), chaque boîtier comprenant une ou plusieurs lampes rayonnantes pour fournir de l'énergie rayonnante à une zone de traitement de l'utilisateur, et chaque boîtier étant relié de manière extensible et flexible à un ou plusieurs autres boîtiers ; et
un boîtier de commande (104) qui abrite un dispositif de commande et un interrupteur à bouton marche/arrêt (105) et qui est relié de manière extensible et flexible à une ou plusieurs sections d'énergie rayonnante, le boîtier de commande étant relié de manière opérationnelle aux boîtiers d'énergie rayonnante et configuré pour commander le fonctionnement des lampes rayonnantes,
les boîtiers étant reliés de manière extensible et flexible de telle sorte qu'ils forment un fer à cheval.

2. Dispositif portable extensible et flexible selon la revendication 1, le boîtier de commande (104) comprenant une source d'énergie permettant le fonctionnement d'une ou plusieurs lampes rayonnantes.

3. Dispositif de photothérapie portable extensible et flexible selon la revendication 1 ou la revendication 2, comprenant en outre :
une paroi réfléchissante extensible et flexible (122) comprenant une pluralité de zones de communication d'énergie rayonnante (483) alignées avec les lampes rayonnantes et disposées de manière à communiquer l'énergie rayonnante à la zone de traitement de l'utilisateur.

4. Dispositif de photothérapie portable extensible et flexible selon l'une quelconque des revendications précédentes, comprenant en outre :
une couche adhésive extensible et flexible (508) comprenant une première sous-surface (510) et une seconde sous-surface (514), la première sous-surface étant fixée de manière amovible au dispositif de photothérapie portable et la seconde sous-surface étant associée de manière opérationnelle à la fixation amovible du dispositif de photothérapie portable à la zone de traitement de l'utilisateur.

5. Dispositif portable extensible et flexible selon l'une quelconque des revendications précédentes, la pluralité de lampes rayonnantes comprenant au moins une des deux énergies rayonnantes de longueur d'onde rouge et infrarouge.

6. Dispositif portable extensible et flexible selon l'une quelconque des revendications précédentes, comprenant en outre un microcontrôleur intégré et portable (108) configuré pour commander le fonctionnement du dispositif de photothérapie.

7. Dispositif portable extensible et flexible selon l'une quelconque des revendications précédentes, une ou plusieurs des parois réfléchissantes (122) et de la couche adhésive (501) étant en matériau à base de silicium et/ou d'uréthane.
